# FASCICULE DE BREVET EUROPEEN

(11) **EP 1 838 653 B1**
(45) Date de publication et mention de la délivrance du brevet: **04.08.2010**
(21) Numéro de dépôt: 06709088.6
(22) Date de dépôt: 13.01.2006
(51) Int. Cl.: C07C 49/607, C07C 35/205, C07C 43/188, C11B 9/00

(54) **NOUVEAUX DERIVES DE TRIMETHYLCYCLODODECATRIENE, LEUR UTILISATION ET PRODUITS PARFUMES LES CONTENANT**
NEUE TRIMETHYLCYCLODODECATRIEN-DERIVATE, VERWENDUNG DAVON UND PARFÜMIERTE ARTIKEL DAMIT
NOVEL TRIMETHYLCYCLODODECATRIENE DERIVATIVES, USE THEREOF, AND PERFUMED PRODUCTS CONTAINING THE SAME

(30) Priorité: 19.01.2005 FR 0500550
(43) Date de publication de la demande: 03.10.2007
(73) Titulaire: V. Mane Fils, 06620 Bar sur Loup (FR)
(72) Inventeur: MANE, Jean, F-06130 Grasse (FR); CHANOT, Jean-Jacques, F-06530 Speracedes (FR); SCHROEDER, Martin, Ashford TN23 5GE (GB)
(74) Mandataire: Cabinet Plasseraud
(86) Numéro de dépôt international: PCT/FR2006/000081
(87) Numéro de publication internationale: WO 2006/077306

(56) Documents cités:
- FR-A- 1 528 158
- US-A- 3 723 478
- DATABASE ZCAPLUS CHEMICAL ABSTRACTS SERVICE, COLUMBUS, OHIO, US; XP002379859 Database accession no. 1989:135534 -& NL 8 700 469 A (NAARDEN INTERNATIONAL) 16 septembre 1988 (1988-09-16)

## Description

La présente invention concerne d'une manière générale de nouveaux composés odorants, qui peuvent être utilisés en parfumerie. L'invention concerne notamment de nouveaux alcools et éthers macrocycliques, leur procédé de synthèse et leur utilisation en parfumerie du fait de leurs propriétés odorantes.

Le terme parfumerie est ici utilisé pour désigner non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires; et de produits d'entretien, tels qu'assouplissants, détergents, lessives, désodorisants d'ambiance, et produits similaires.

Le terme odorant est utilisé ici pour désigner un composé qui exhale une odeur.

Des macrocycles sont déjà utilisés en parfumerie. En particulier, la trimérisation de l'isoprène résulte en un mélange d'alcènes macrocycliques, qui contient principalement deux stéréo-isomères aux rapports variés, notamment le 1,5,10-trimé-thyl-cyclododéca-1,5,9-triène et le 1,5,9-triméthyl-cyclododéca-1,5,9-triène. Ce mélange de macrocycles fournit l'industrie de la parfumerie avec une matière première à faible prix. Certains dérivés, tels que le Cedroxyde^{™} (triméthyl-13-oxabicyclo[10.1.0]tridéca-4,8-diène de chez Firmenich), le Boisanol^{™} de chez Symrise ou le Trimofix^{™} O (2,5,10-triméthyl-2,5,9-cyclododécatrièn-1-yl méthyl cétone) de chez IFF, pour n'en citer que quelques-uns, sont utilisés couramment.

Le brevet néerlandais NL 8700469 décrit des dérivés de 4,8-cyclododécadiénol ayant une odeur de patchouli.

Par ailleurs, le brevet US 3,723,478 de Firmenich décrit l'oxydation de certains triméthylcyclododécatriènes en dérivés cétoniques. Les triméthyl-cyclododécadiénones sont obtenues par une époxydation, suivie de l'ouverture de l'époxyde puis d'une oxydation. Cependant, les rendements d'étapes de cette voie de synthèse sont faibles et les transformations ne sont pas très sélectives.

Par conséquent, il subsiste un besoin pour de nouveaux macrocycles odorants, et pour un procédé de synthèse dont le rendement et la sélectivité soient améliorés.

Les inventeurs ont découvert de façon surprenante un nouveau procédé de synthèse de nouveaux macrocycles, qui ont des propriétés odorantes et qui peuvent donc être utilisés en parfumerie.

La présente invention a donc pour objet une nouvelle famille de macromolécules cycliques représentée par la formule suivante (I) : 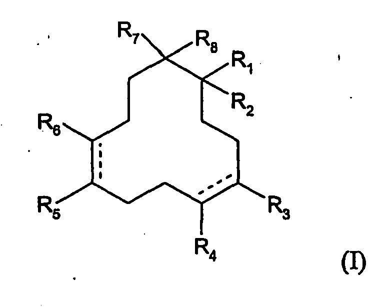 dans laquelle :
A)
   a) R₄, R₅, R₇ représentent chacun un atome d'hydrogène et R₃, R₆ et R₈ représentent chacun un radical méthyle, ou
   b) R₄, R₆, R₇ représentent chacun un atome d'hydrogène et R₃, R₅ et R₈ représentent chacun un radical méthyle, ou
   c) R₃, R₆ et R₇ représentent chacun un atome d'hydrogène et R₄, R₅ et R₈ représentent chacun un radical méthyle,
   et
   les liaisons en pointillé sont présentes et représentent des doubles liaisons cis- ou trans- et R₁ représente un atome d'hydrogène et R₂ représente un groupe -OH, -OCH₃ ou -OC₂H₅,
   ou
   les liaisons en pointillé sont absentes et R₁ représente un atome d'hydrogène et R₂ représente un groupe -OCH₃ ou -OC₂H₅,
   ou
B) R₁, R₄, R₆ représentent chacun un atome d'hydrogène et R₂, R₃ et R₅ représentent chacun un radical méthyle,
   et
   les liaisons en pointillé sont présentes et représentent des doubles liaisons *cis* ou *trans* et R₇ représente un atome d'hydrogène et R₈ représente un groupe -OH, -OCH_{3,}-OC₂H₅,
   ou
   les liaisons en pointillé sont absentes et R₇ représente un atome d'hydrogène et R₈ représente un groupe -OCH₃ ou -OC₂H₅.

En particulier, l'invention a pour objet les nouveaux composés de formule (5a-d), (6a-d), (6a'-d') ci-dessous, 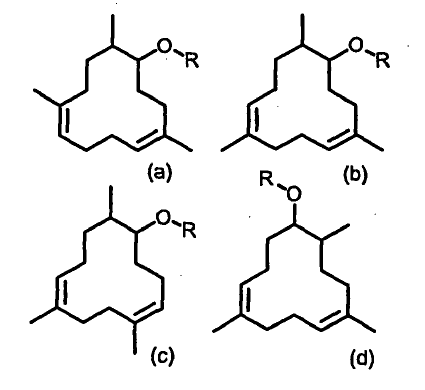 dans laquelle R est un atome d'hydrogène (composés 5a, 5b, 5c, 5d) ou un groupe méthyle (composés 6a, 6b, 6c, 6d) ou un groupe éthyle (composés 6a', 6b', 6c', 6d').

Les composés 5a, 6a, 6a' sont des composés de formule (I) où
R₁ représente un atome d'hydrogène,
R₂ représente un groupe -OH (composé 5a), -OCH₃ (composé 6a), ou -OC₂H₅ (composé 6a'),
R₃ est un groupe méthyle,
R₄ est un atome d'hydrogène,
R₅ est un atome d'hydrogène,
R₆ est un groupe méthyle,
R₇ est un atome d'hydrogène,
R₈ est un groupe méthyle, et
les doubles liaisons sont présentes.

Les composés 5b, 6b, 6b' sont des composés de formule (I) où
R₁ représente un atome d'hydrogène,
R₂ représente un groupe -OH (composé 5b), -OCH₃ (composé 6b), ou
-OC₂H₅(composé 6b'),
R₃ est un groupe méthyle,
R₄ est un atome d'hydrogène,
R₅ est un groupe méthyle,
R₆ est un atome d'hydrogène,
R₇ est un atome d'hydrogène,
R₈ est un groupe méthyle, et
les doubles liaisons sont présentes.

Les composés 5c, 6c, 6c' sont des composés de formule (I) où
R₁ représente un atome d'hydrogène,
R₂ représente un groupe -OH (composé 5c), -OCH₃ (composé 6c), ou
-OC₂H₅(composé 6c'),
R₃ est un atome d'hydrogène,
R₄ est un groupe méthyle,
R₅ est un groupe méthyle,
R₆ est un atome d'hydrogène,
R₇ est un atome d'hydrogène,
R₈ est un groupe méthyle, et
les doubles liaisons sont présentes.

Les composés 5d, 6d, 6d' sont des composés de formule (I) où
R₁ représente un atome d'hydrogène,
R₂ est un groupe méthyle,
R₃ est un groupe méthyle,
R₄ est un atome d'hydrogène,
R₅ est un groupe méthyle,
R₆ est un atome d'hydrogène,
R₇ est un atome d'hydrogène,
R₈ représente un groupe -OH (composé 5d), -OCH₃ (composé 6d), ou -OC₂H₅, (composé 6d'), et
les doubles liaisons sont présentes.

En particulier, l'invention a également pour objet les nouveaux composés de formule (7a-d) (7a'-d') ci-dessous, 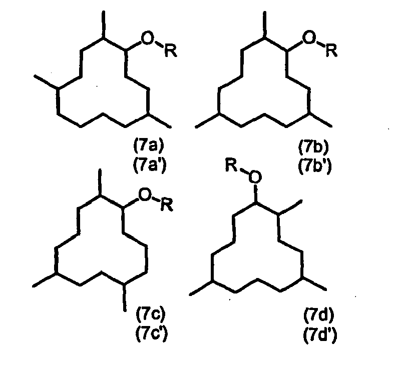 dans laquelle R est un groupe méthyle (composés 7a, 7b, 7c, 7d) ou un groupe éthyle (composés 7a', 7b', 7c', 7d').

Les composés 7a, 7a' sont des composés de formule (I) où
R₁ représente un atome d'hydrogène,
R₂ représente un groupe -OCH₃ (composé 7a), ou -OC₂H₅, (composé 7a')
R₃ est un groupe méthyle,
R₄ est un atome d'hydrogène,
R₅ est un atome d'hydrogène,
R₆ est un groupe méthyle,
R₇ est un atome d'hydrogène,
R₈ est un groupe méthyle, et
les doubles liaisons sont absentes.

Les composés 7b, 7b' sont des composés de fonnule (I) où
R₁ représente un atome d'hydrogène,
R₂ représente un groupe -OCH₃ (composé 7b), ou -OC₂H₅, (composé 7b')
R₃ est un groupe méthyle,
R₄ est un atome d'hydrogène,
R₅ est un groupe méthyle,
R₆ est un atome d'hydrogène,
R₇ est un atome d'hydrogène,
R₈ est un groupe méthyle, et
les doubles liaisons sont absentes.

Les composés 7c, 7c' sont des composés de formule (I) où
R₁ représente un atome d'hydrogène,
R₂ représente un groupe -OCH₃ (composé 7c), ou -OC₂H₅, (composé 7c')
R₃ est un atome d'hydrogène,
R₄ est un groupe méthyle,
R₅ est un groupe méthyle,
R₆ est un atome d'hydrogène,
R₇ est un atome d'hydrogène,
R₈ est un groupe méthyle, et
les doubles liaisons sont absentes.

Les composés 7d, 7d' sont des composés de formule (I) où
R₁ représente un atome d'hydrogène,
R₂ est un groupe méthyle,
R₃ est un groupe méthyle,
R₄ est un atome d'hydrogène,
R₅ est un groupe méthyle,
R₆ est un atome d'hydrogène,
R₇ est un atome d'hydrogène,
R₈ représente un groupe -OCH₃ (composé 7d), ou -OC₂H₅, (composé 7d'), et
les doubles liaisons sont absentes.

Les composés de formule (I) peuvent être présents sous forme d'un isomère ou d'un mélange d'isomères, en particulier d'un énantiomère ou d'un mélange d'énantiomères, ou d'un mélange racémique, ou d'un diastéréoisomère ou mélange de diastéréoisomères.

Les composés de formule (I) ont tous des propriétés odorantes. Les composés (5a-d) ont une odeur ambrée, musquée. Les composés (6a-d) et (6a'-d') présentent des notes boisées, camphrées de registre vétiver. Les composés (7a-d) possèdent des notes fruitées, vertes. De part ces propriétés odorantes, ces différents produits, tout particulièrement les éthers méthyliques (6a-d), trouvent un emploi très varié, notamment en parfumerie.

L'invention a donc également pour objet l'utilisation de ces composés comme agents odorants.

La présente invention a également pour objet le procédé de synthèse des composés de formule (I).

Chacun des composés (5a-d), (6a-d), (6a'-d'), (7a-d) et (7a'-d') peut être synthétisé directement ou indirectement à partir des composés (4a-d) suivants : 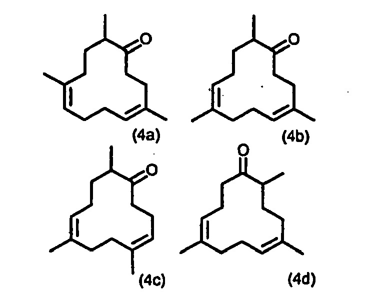 qui peuvent eux-mêmes être synthétisés en partant de triméthyl-cyclododécatriènes. La présente invention fournit également un procédé nouveau de préparation des cétones macrocycliques 4a-d, décrit ci-après.

Le schéma suivant représente un procédé de synthèse des composés selon l'invention.

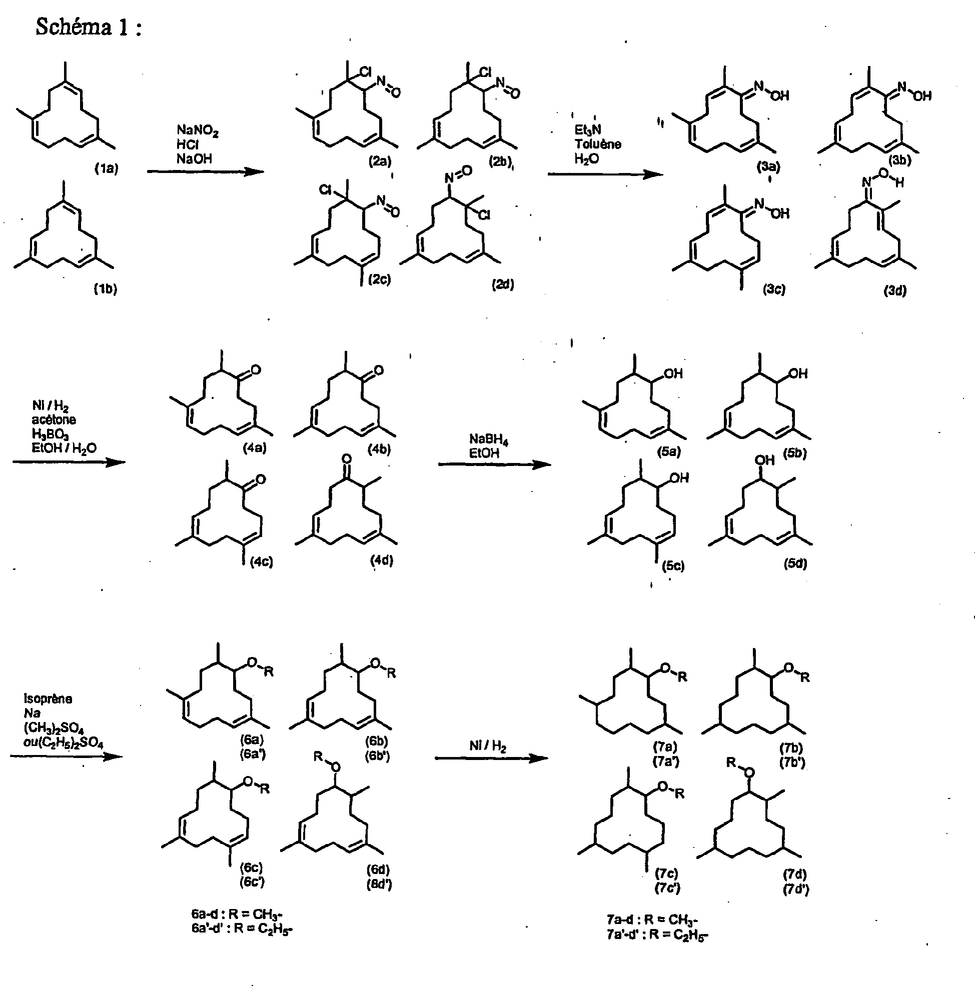

D'une manière générale, le procédé selon l'invention comprend les étapes suivantes :
- formation de dérivés chloro-nitroso à partir du 1,5,10-triméthyl-cyclododéca-1,5,9-triène (1a) et du 1,5,9-triméthyl-cyclododéca-1,5,9-triène (1b),
- transformation de ces dérivés en oximes,
- transformation réductrice des oximes en cétones en présence de Nickel de Raney, d'acétone et d'acide borique,
- réduction des cétones en alcools,
- éthérification en éthers,
- éventuellement hydrogénation en éthers saturés.

Dans un premier temps, les dérivés chloro-nitroso (2a-d) des 1,5,10-triméthylcyclododécatriène (1a) et 1,5,9-triméthylcyclododécatriène (1b) sont formés. Il existe un grand nombre de réactifs disponibles pour cette réaction, connus de l'homme du métier, et qui permettent d'aboutir au résultat attendu. Parmi ces réactifs, le nitrite de sodium est intéressant pour des raisons économiques. Les triméthylcyclododécatriènes (1a-b) sont refroidis, dans un solvant comme le sec-butanol, à une température de l'ordre de -10°C à -20°C, car la réaction est très exothermique. Le nitrite de sodium (réactif utilisé dans ce mode de réalisation) est ensuite ajouté en petites portions pendant que l'acide chlorhydrique est parallèlement ajouté goutte à goutte. Des cristaux blancs se forment. Après la fin des additions des réactifs, le milieu réactionnel est encore agité pendant environ 16 heures en laissant remonter la température dans la masse à température ambiante. Ensuite, la solution est neutralisée sous refroidissement, de manière à ce que la température n'excède pas 25°C dans la masse, avec de la soude ou une autre base adéquate. Les dérivés chloro-nitroso (2a-d) sont obtenus sous forme de cristaux, qui sont lavés, filtrés, séchés, et ils peuvent être directement engagés dans l'étape suivante.

Ensuite, les dérivés chloro-nitroso (2a-d) sont transformés en oximes (3a-d), dans des conditions appropriées que l'homme du métier peut déterminer compte tenu de ses connaissances générales. La transformation en oximes se fait de préférence dans un milieu aqueux en présence d'une base. L'utilisation de plusieurs bases est envisageable, cependant un bon rendement est obtenu en présence de triéthylamine. Les cristaux (2a-d) sont mis en suspension dans un mélange d'eau, de toluène et de triéthylamine. La suspension est chauffée au reflux pendant environ quatre à six heures. La disparition complète des cristaux indique une transformation totale. Après avoir été refroidies, les phases sont séparées et la phase organique est lavée à neutre avec de l'eau salée, puis del'acide sulfurique, et encore de l'eau salée. Après concentration du solvant, les oximes (3a-d) sont obtenues sous forme d'une masse brune, qui peut être directement engagée dans la réaction suivante, car la purification est possible mais difficile compte tenu de la masse moléculaire et de la complexité du produit brut.

Il existe une grande variété de méthodes oxydantes et réductrices pour la transformation d'une oxime en cétone, connues de l'homme du métier, et plusieurs réactifs peuvent être utilisés pour obtenir des cétones, comme le bisulfite de sodium, le sulfate de cuivre, le nitrite sodium, l'acide levulinique et l'oxone, pour n'en citer que quelques-uns. Dans le cas présent, tous ces essais se sont soldés par des échecs. Seule la transformation réductrice en présence de Nickel de Raney, d'acétone/éthanol et d'acide borique a permis l'obtention des cétones (4a-d). Pour cela, les oximes (3a-d) ont été partiellement dissoutes dans un mélange d'éthanol et d'acétone et ont été placées dans un autoclave en présence de Nickel de Raney et d'acide borique. Le milieu réactionnel est ensuite agité pendant environ 14 heures à une température de l'ordre de 25°C à 60°C et une pression d'hydrogène de l'ordre de 1×10⁴ HPa et 5×10⁴ HPa, ce qui permet l'obtention des cétones (4a-d), à savoir la 4,9,12-triméthyl-cyclododeca-4,8-diénone (4a); la 4,8,12-triméthyl-cyclododeca-4,8-diénone (4b) ; la 5,8,12-triméthyl-cyclododeca-4,8-diénone (4c) ; la 5,9,12-triméthyl-cyclododeca-4,8-diénone (4d).

La solution est ensuite refroidie à température ambiante, noyée dans l'eau et extraite avec un solvant comme le toluène par exemple. Les phases organiques réunies sont lavées à neutre, séchées, concentrées puis distillés. Le mélange de cétones (4a-d) possède une odeur musquée, fleurie et légèrement boisée.

La réduction des cétones (4a-d) en alcools (5a-d) peut être faite dans des conditions appropriées que l'homme du métier peut déterminer. En particulier, on peut effectuer la réduction dans l'éthanol en utilisant le borohydrure de sodium, préféré à d'autres réactifs appropriés pour sa facilité d'emploi et son prix raisonnable. Le borohydrure de sodium est ajouté à la solution alcoolique pendant une période d'environ 36 heures à une température d'environ 5°C à 15°C. L'excès de l'hydrure est détruit avec de l'acétone. Après acidification à l'acide chlorhydrique la solution est noyée avec de l'eau puis extraite. Les phases organiques unies sont lavées à neutre, séchées, filtrées et concentrés. Après distillation, les alcools (5a-d) sont obtenus.

Ces alcools possèdent une odeur ambrée, musquée, qui est relativement faible mais réelle.

Les alcools peuvent ensuite être étherifiés dans des conditions appropriées connues de l'homme du métier. Deux classes de réactifs sont en général employées pour l'éthérification des alcools : les alkyles halogénés et les sulfates d'alkyles. Les deux classes de réactifs sont utilisables pour obtenir les éthers (6a-d et 6a'-d'). Pour des raisons économiques, le sulfate de diméthyle et le sulfate de diéthyle sont préférés. Afin de faciliter la formation des alcoolates, de l'isoprène est ajouté goutte à goutte à la solution des triméthyl-cyclododécadiénols (5a-d) dans le tetrahydrofuranne dans laquelle des morceaux de sodium sont mis en suspension. Le sodium réagit d'abord avec l'isoprène en formant un cation qui réagit ensuite rapidement avec les alcools présents. Cette réaction est faite à une température d'environ 10°C à 20°C. Une fois que tout le sodium a réagi, le réactif d'éthérification (sulfate de diméthyl ou diéthyl par exemple) est ajouté. Le milieu réactionnel est mis au reflux pendant environ six heures. La réaction est terminée une fois que la solution est devenue incolore. Une solution aqueuse d'ammoniaque est ajoutée en refroidissant (10°C à 20°C) pour détruire l'excès du sulfate. La phase aqueuse est extraite avec un solvant comme le t-butyl-méthyl éther. Les phases organiques réunies sont lavées à neutre, séchées, filtrées et concentrées. Une distillation fournit les éthers méthyliques (6a-d) ou éthyliques (6a'-d') qui sont des composés nouveaux. Les éthers méthyliques (6a-d) possèdent des notes boisées, camphrées de registre vétiver qui sont très appréciées parmi les parfumeurs. Les éthers éthyliques (6a'-d') possèdent à peu près les mêmes notes que les éthers méthyliques (6a-d), tout en étant beaucoup moins volatils.

Les éthers (6a-d) peuvent ensuite être hydrogénés pour aboutir aux éthers saturés correspondants (7a-d), dans des conditions pouvant être déterminées par l'homme du métier. En particulier, l'hydrogénation peut se faire en présence de palladium sur charbon par exemple, à une température de l'ordre de 20°C à 80°C, sous une pression de l'ordre de 2×10⁴ HPa à 8×10⁴ HPa d'hydrogène. Les éthers obtenus sont des composés nouveaux. Ils possèdent des notes fruitées, vertes mais fugaces.

Le procédé décrit ci-dessus permet d'obtenir chaque groupe de composés 4, 5, 6 et 7 sous forme de mélange a-d ou a'-d'. Il est difficile mais possible de séparer les composés d'un mélange, par des techniques appropriées connues de l'homme du métier, en particulier par séparation sur colonne chirale ou chromatographie préparative. Compte tenu de ces difficultés, il n'est pas forcément nécessaire de les séparer, et les composés peuvent également être utilisés en mélange, puisque chaque mélange possède lui-même des propriétés odorantes.

L'invention a également pour objet l'utilisation d'au moins un composé de formule (I) selon l'invention comme agent odorant, comme agent de masquage d'odeur ou comme agent de neutralisation d'odeur, seul ou en en mélange avec un ou plusieurs autres composés odorants connus de l'homme du métier, que l'homme du métier est à même de choisir en fonction de l'effet recherché. Le ou les agents odorants supplémentaires peuvent être des composés de formule (I) ou d'autres agents odorants connus de l'homme du métier.

L'invention a également pour objet les compositions comprenant un produit de base et une quantité efficace d'un ou plusieurs composés de formule (I) selon l'invention.

Il peut s'agir d'une composition elle-même odorante, ou d'une composition dans laquelle l'agent odorant est utilisé pour masquer ou neutraliser certaines odeurs.

Le produit de base sera aisément déterminé par l'homme du métier en fonction de la composition envisagée et donc de l'utilisation envisagée, pour lesquelles les composants habituels, tels que solvant(s) et/ou adjuvant(s), sont bien connus.

La quantité efficace des composés de formule (I) selon l'invention incorporé dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier 0,1 à 50% en poids, notamment 0,1 à 30% en poids. Les composés de formule (I) selon l'invention peuvent être utilisés tels quels ou ils peuvent être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a donc aussi pour objet l'utilisation des composés de formule (I) pour la préparation d'une composition odorante ou d'un article odorant dans les applications décrites ci-dessus, en particulier en parfumerie, en cosmétique, par exemple pour des shampooings ou des savons, et pour des produits d'entretien, tels que des assouplissants ou des lessives.

L'invention concerne en particulier une composition de parfumerie, notamment une base ou un concentré parfumant, une eau de Cologne, une eau de toilette ou un parfum, comprenant au moins un composé de formule (I) ou une composition comprenant au moins un composé de formule (I).

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade comprenant au moins un composé de formule (I) ou au moins une composition comprenant au moins un composé de formule (I). Un autre objet de l'invention est une méthode de traitement ou de soin cosmétique, préventive ou non, mettant en oeuvre au moins un composé de formule (I) ou au moins une composition comprenant au moins un composé de formule (I).

L'invention concerne encore un produit d'entretien, notamment assouplissant, détergent, lessive, désodorisant d'ambiance, comprenant au moins un composé de formule (I) ou au moins une composition comprenant au moins un composé de formule (I).

Les composés selon l'invention peuvent être utilisé, seuls ou en combinaison, tels quels ou être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a donc aussi pour objet l'utilisation des composés nouveaux pour la préparation d'une composition odorante ou d'un article odorant dans les applications décrites ci-dessus, en particulier en parfumerie, en cosmétique, par exemple pour des shampooings ou des savons, et pour des produits d'entretien, tels que des assouplissants ou des lessives.

L'invention concerne en particulier une composition de parfumerie, notamment une base ou un concentré parfumant, une eau de Cologne, une eau de toilette ou un parfum, comprenant au moins un composé de formule (I) ou une composition comprenant au moins un composé de formule (I).

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade, comprenant au moins un composé de formule (I) ou une composition comprenant au moins un composé de formule (I). Un autre objet de l'invention est une méthode de traitement ou de soin cosmétique, préventive ou non, mettant en oeuvre au moins un composé de formule (I) ou au moins une composition comprenant au moins un composé de formule (I).

Les exemples suivants illustrent davantage les différents procédés de fabrication des molécules déjà connues ou nouvelles selon l'invention, ainsi que leur utilisation et leur intérêt. Ces exemples ne sont présentés que dans un but d'illustration et ne doivent pas être considérés comme limitatifs de l'invention.

### Exemple 1 : Synthèse des chloro-tnméthvl-nitroso-cvclododécadiènes (2a-d)

Dans un ballon de quatre litres muni d'une tubulure latérale avec entonnoir à poudre, d'une ampoule d'introduction et d'un thermomètre, on charge 408.0 g (2.00 mol) de triméthyl-cyclododécatriènes (1a et b) et 700.0 g de sec-butanol. La solution est refroidie entre -10°C et -15°C. On introduit simultanément sur une période d'environ six heures 640.0 g (6.00 mol) d'acide chlorhydrique à 34 % goutte à goutte et 160.0 g (2.32 mol) de nitrite de sodium en portions. La réaction est très exothermique. Un refroidissement efficace est conseillé pour ne pas dépasser 0°C dans la masse. Le milieu réactionnel est encore agité pendant seize heures en laissant la température dans la masse remonter à température ambiante. Des cristaux blancs, gris se forment et le milieu réactionnel devient pâteux. Ensuite, 1000.0 g d'eau sont ajouté pour faciliter l'agitation. On refroidit à nouveau et on introduit 300.0 g (3.53 mol) de soude à 47 % goutte à goutte sans dépasser 25°C dans la masse. Les cristaux sont filtrés, rincés avec 325.0 g d'hexane et tamisés (tamis 1.25). On sèche les cristaux sous vide (d'abord sous 40 HPa puis sous 0.7 HPa). On obtient 419.7 g (max. 1.56 mol) de chloro-triméthyl-nitroso-cyclododécadiènes (2a-d), qui contiennent des traces de produit de départ et qui sont pratiquement insolubles dans tous les solvants essayés. Le rendement est maximal à 78.0 %. Les cristaux sont directement engagés dans l'étape suivante.

### Exemple 2 : Synthèse des triméthyl-cyclododécatiiénone oximes (3a-d)

Dans un ballon de deux litres, muni d'un réfrigérant, d'une ampoule d'introduction et d'un thermomètre, on introduit 270.0 g (1.00 mol) de chloro-triméthyl-nitroso-cyclododécadiènes (2a-d), 301.0 g de toluène et 121.5 g (1.20 mol) de triéthyl amine et on porte le mélange à 60°C. On ajoute 350.0 ml d'eau goutte à goutte à cette température, puis on porte le milieu réactionnel au reflux (environ 80°C) en agitant fortement pendant quatre heures. Le milieu réactionnel est refroidi à 60°C et la phase aqueuse est séparée. La phase organique est ensuite lavée deux fois avec 200 ml d'eau salée, une fois avec 250 ml d'acide sulfurique à 10% puis encore une fois avec 200 ml d'eau salée. On concentre le solvant sous pression réduite (environ 40 HPa) sans dépasser 50°C dans la masse. On obtient 228.00 g d'une masse pâteuse, brune qui est engagée directement dans l'étape suivante.

### Exemple 3: Synthèse de triméthyl-cyclododécadiénones (4a-d)

On charge dans un autoclave d'une capacité de 800 ml 78.0 g d'éthanol, 158.0 g (2.72 mol) d'acétone, 2.3 g (0.04 mol) d'acide borique, 30.0 g (1.67 mol) d'eau, 4.0 g de Nickel de Raney et 228.0 g de triméthyl-cyclododécatriénone oximes (3a-d) brutes. L'appareil est purgé trois fois avec l'hydrogène. On agite pendant 24 heures à 50°C et une pression d'hydrogène de 3×10⁴ HPa. L'appareil est refroidi à température ambiante et le milieu réactionnel est récupéré. On ajoute 500 ml de toluène, puis 300 ml d'eau salée. Les phases sont séparées et la phase aqueuse est extraite une fois avec 100 ml de toluène. Les phases organiques réunies sont lavées six fois avec 100 ml d'acide chlorhydrique à 10% jusqu'à pH neutre. On lave encore une fois avec 100 ml d'eau salée, puis on sèche, filtre et concentre sous vide (40 HPa) sans dépasser 50°C dans la masse. Après une distillation, on obtient 124.8 g (0.57 mol) de triméthyl-cyclododécadiénones (4a-d) (Téb. : 94 -97°C/0.3 HPa). Le rendement sur les deux étapes est à 57%. Les analyses des spectres infra rouges, RMN et de masse correspondent aux structures des composés attendus.

### Exemple 4 : Synthèse de trimétyl-cyclododécadiénols (5a-d)

On place dans un ballon de deux litres muni d'un thermomètre 124.8 g (0.57 mol) de triméthyl-cyclododécadiénones (4a-d) et 390.0 g d'éthanol. On refroidit à 5°C et on ajoute 10.7 g de borohydrure de sodium en petites portions sans dépasser 10°C dans la masse. Le mélange est agité à cette température pendant 42 heures. Des quantités supplémentaires de borohydrure de sodium sont ajoutées pendant cette période (5.3 g après six heures, 10.1 g après 24 heures et 5.3 g après 36 heures; quantité totale de borohydrure de sodium engagée : 31.4 g (0.863 mol)). On ajoute goutte à goutte 79.0 g (1.36 mol) d'acétone pour détruire l'excès du réducteur sans dépasser 10°C dans la masse. Le milieu réactionnel est acidifié avec 500.0 g d'acide chlorhydrique à 10% sans dépasser 10°C dans la masse. On ajoute encore 800 g d'eau, puis 344 g de toluène sous forte agitation. Les phases sont séparées et la phase organique est lavée trois fois avec 200 g d'eau et une fois avec 200 g d'eau salée. On sèche, filtre et évapore le toluène sous pression réduite (40 PHa) sans dépasser 50°C dans la masse. On obtient après distillation 69.6 g (0.31 mol) de triméthyl-cyclododécadiènes (5a-d) (Téb. : 98-100°C/0.2 HPa). Le rendement est à 55.3%. Les analyses des spectres infra rouges, RMN et de masse correspondent aux structures des composés attendus.

### Exemple 5 : Synthèse des méthoxy-triméthyl-cyclododécadiènes (6a-d)

Placer dans un ballon de deux litres muni d'un réfrigérant, d'un thermomètre et d'une ampoule d'introduction sous azote 360.0 g de tetrahydrofuranne, 69.6 g (0.31 mol) de triméthyl-cyclododécadiénols (5a-d) et 8.2 g 0.36 mol) de sodium, coupé en petits morceaux. On refroidit le milieu réactionnel à 15°C et ajoute 41.6 ml (28.3 g, 0.42 mol) d'isoprène goutte à goutte sans dépasser 15°C dans la masse. L'agitation est continuée jusqu'à la dissolution complète du sodium. On refroidit à 10°C et on ajoute 33.8 ml (45.0 g, 0357 mol) de diméthyl sulfate goutte à goutte à cette température. On porte le milieu réactionnel au reflux pendant 6 heures jusqu'à la décoloration totale de la solution. On refroidit à 10°C et on ajoute goutte à goutte 300 ml d'une solution aqueuse d'ammoniaque à 10% sans dépasser 15°C dans la masse. Les phases sont séparées et la phase aqueuse est extraite deux fois avec 150 ml de t-butyl-méthyl-éther. Les phases organiques réunies sont lavées une fois avec 150 g d'acide chlorhydrique à 10 %, deux fois avec 150 g d'eau puis une fois avec 150 g d'eau salée. On sèche, filtre et évapore les solvants sous vide (40 HPa) sans dépasser 50°C dans la masse. On obtient après distillation 33.5 g (0.14 mol) de méthoxy-triméthyl-cyclododécadiènes (6a-d) (Téb. : 68-72°C/0.2 HPa). Le rendement est à 45.7%. Les analyses des spectres infra rouges, RMN et de masse correspondent aux structures des composés attendus.

### Exemple 6 : Synthèse des éthoxy-triméthyl-cyclododécadiènes (6a'-d')

Les éthoxy-triméthyl-cyclododécadiène (6a'-d') sont synthétisés comme décrit dans l'exemple 4, en engageant 28.9g (0.12 mol) de triméthyl-cyclododécadiènes (5a-d) et 20.4 g (0.36 mol) de diéthyl sulfate. On obtient 18.2 g (0.07 mol) d'éthoxy-triméthyl-cyclododécadiènes (6a'-d') (Téb. : 105-108°C/0.2 HPa). Le rendement est à 62.7%. Les analyses des spectres infra rouges, RMN et de masse correspondent aux structures des composés attendus.

### Exemple 7 : Synthèse des méthoxy-triméthyl-cyclododécanes (7a-d)

On place dans un autoclave 7.6 g 32 (mmol) de méthoxy-triméthyl-cyclododécadiènes (6a-d), 100 ml d'éthanol et 0.5 g de palladium sur charbon. L'appareil est fermé et purgé trois fois à l'hydrogène. On agite pendant six heures à 40°C et 4×10⁴ HPa d'hydrogène, puis on refroidit à température ambiante et récupère le milieu réactionnel. Le catalyseur est filtré et le solvant est évaporé sous pression réduite (40 HPa) sans dépasser 50°C dans la masse. Après micro-distillation, on obtient 5.0 g (20.8 mmol) de méthoxy-triméthyl-cyclododécanes (7a-d) (71-78°C/0.3 HPa). Le rendement est à 65.0%. Les analyses des spectres infra rouges, RMN et de masse correspondent aux structures des composés attendus.

### Exemple 8 : Evaluation olfactive

Dans un premier temps, les caractéristiques odorantes des méthoxy-triméthyl-cyclododécadiènes (6a-d) purs ont été évaluées par un panel en même temps que les caractéristiques odorantes de composés connus présents sur le marché. Le panel d'évaluation est composé de plusieurs professionnels, évaluant qualitativement et quantitativement chaque composé. Le mélange de composés a été décrit comme boisé, camphré, dans le registre de vétyver et très puissant. Des analogies ont été trouvées avec certains produits de marché, comme le Spirambrène^{™} (2,2,3',7',7'-Pentamethylspiro-1,3-dioxan-5,2'-norcarane) de chez Givaudan, le Karanal^{™} (5-sec-Butyl-2-(2,4-dimethyl-cyclohex-3-enyl)-5-methyl-[1,3]dioxane) de chez Quest International, le Cédramber^{™} (6-Methoxy-3,6,8,8-tetramethyl-octahydro-3a,7-methano-azulene) de chez International Flavours and Fragrances ou le Boisambrène^{™} (Ethoxy-méthoxy-cyclododécane) de chez Henkel.

Ensuite, deux compositions alcooliques ont été mises au point, dans lesquelles le mélange de méthoxy-triméthyl-cyclododécadiènes (6a-d) a été comparé avec des produits commerciaux. Dans chaque cas, les évaluations de l'impact olfactif ont été effectuées à t₀, t₊₄₈ₕ, t₊₁₆₈ₕ pour évaluer les notes de tête, de coeur et de fond, en comparaison avec des composés connus.

### Composition n° 1

| | Essai 1 | Essai 2 | Essai 3 |
|---|---|---|---|
| Composant | % (en poids) | % | % |
| Acétate de cédryle cristallisé ¹⁾ | 0.80 | 0.80 | 0.80 |
| Acétate de Linalyle | 1.00 | 1.00 | 1.00 |
| Acétate de Vétyveryle Haïti | 4.50 | 4.50 | 4.50 |
| Trans Anéthol | 0.30 | 0.30 | 0.30 |
| Orcanox ²⁾ | 0.40 | 0.40 | 0.40 |
| Bergamote Ess. | 0.60 | 0.60 | 0.60 |
| Orange douce Ess. | 0.30 | 0.30 | 0.30 |
| Cashmeran ³⁾ | 0.70 | 0.70 | 0.70 |
| Galaxolide ⁴⁾ | 20.00 | 20.00 | 20.00 |
| Dihydro jasmonate de méthyle ⁵⁾ | 6.00 | 6.00 | 6.00 |
| Iso E Super ⁶⁾ | 52.00 | 52.00 | 52.00 |
| Linalol | 0.50 | 0.50 | 0.50 |
| Orange Terpènes Brésil | 0.10 | 0.10 | 0.10 |
| Vetyver Ess. Haïti | 1.00 | 1.00 | 1.00 |
| Vetyverol Haïti ¹⁾ | 1.00 | 1.00 | 1.00 |
| Acétate d'isoeugényle 10 % dans DPG⁷⁾ | 0.10 | 0.10 | 0.10 |
| Cardamome Ess. | 0.40 | 0.40 | 0.40 |
| Citral pur du Litsea Cubeba 10 % dans DPG ⁷⁾ | 0.30 | 0.30 | 0.30 |
| Cédramber ⁸⁾ | 10.00 | / | / |
| Boisambrene ⁹⁾ | / | / | 10.00 |
| Méthoxy-triméthyl-cyclododécadiène (6a-d) | / | 10.00 | / |
| Total | 100.00 | 100.00 | 100.00 |

| | | | |
|---|---|---|---|
| 1. Origine : V. Mane Fils, France 2. 3a,6,6,9a-Tetramethyl-dodecahydro-naphtho[2,1-*b*]furan; origine : V. Mane Fils, France. 3. 1,1,2,3,3-Pentamethyl-1,2,3,5,6,7-hexahydro-inden-4-one ; origine : International Flavours and Fragrances, USA. 4. 1,1,2,3,3,8-Hexamethyl-1,2,3,5,7,8-hexahydro-6-oxa-cyclopenta[b]naphthalène ; origine : International Flavours and Fragrances, USA. 5. [3-Oxo-2-((E)-pentyl)-cyclopentyl]-acetic acid methyl ester ; origine : Firmenich, Switzerland. 6. 1-(2,3,8,8-Tetramethyl-1,2,3,4,5,6,7,8-octahydro-naphthalen-2-yl)-ethanone ; origine : International Flavours and Fragrances, USA. 7. Dipropylene glycol. 8. 6-Methoxy-3,6,8,8-tetramethyl-octahydro-3a,7-methano-azulene ; origine : International Flavours and Fragrances, USA. 9. Ethoxy-méthoicy-cyclododécane ; origine : Henkel, Allemagne. | | | |

### Composition n° 2

| | Essai 4 | Essai 5 | Essai 6 |
|---|---|---|---|
| Matière Engagée | Parts (en poids) | parts | parts |
| Violettyne ¹⁾ | 5 | 5 | 5 |
| Orcanox ²⁾ | 20 | 20 | 20 |
| Calon 1951 Cal ³⁾ | 5 | 5 | 5 |
| Veramoss ⁴⁾ | 2 | 2 | 2 |
| Ethyl linalol ⁵⁾ | 50 | 50 | 50 |
| Florol ⁶⁾ | 10 | 10 | 10 |
| Dihydro jasmonate de méthyle ⁷⁾ | 287 | 287 | 287 |
| Helional ⁸⁾ | 30 | 30 | 30 |
| Iso E Super ⁹⁾ | 20 | 20 | 20 |
| Lilial ¹⁰⁾ | 10 | 10 | 10 |
| Melonal ¹¹⁾ | 1 | 1 | 1 |
| Methylionanthème ¹²⁾ | 5 | 5 | 5 |
| Salicylate de benzyle | 10 | 10 | 10 |
| Acétate de cis-3 hexényle ¹³⁾¹⁴⁾ | 2 | 2 | 2 |
| Acétate de styrallyle ¹⁴⁾ | 5 | 5 | 5 |
| Allyle amyle glycolate ¹⁴⁾ | 1 | 1 | 1 |
| Galbex ¹⁵⁾ | 2 | 2 | 2 |
| Cis-3 hexénol¹⁴⁾ | 2 | 2 | 2 |
| Liffarome ¹⁶⁾ | 2 | 2 | 2 |
| Triplal ¹⁴⁾¹⁷⁾ | 1 | 1 | 1 |
| Méthoxy-triméthyl-cyclododécadiène (6a-d) | / | 30 | / |
| Boisambrène forte ¹⁸⁾ | / | / | 30 |
| Total | 470 | 500 | 500 |

| | | | |
|---|---|---|---|
| 1. (E)-Undeca-1,3-dien-5-yne ; origine : Firmenich, Switzerland 2. 3a,6,6,9a-Tetramethyl-dodecahydro-naphtho[2,1-*b*]furan; origine : V. Mane Fils, France. 3. 7-Methyl-benzo[b][1,4]dioxepin-3-one ; origine : Symrise, Allemagne. 4. 2,4-Dihydroxy-3,6-dimethyl-benzoic acid methyl ester ; origine : International Flavours and Fragrances, USA. 5. Origine : Givaudan, Switzerland. 6. 2-Isobutyl-4-methyl-tetrahydro-pyran-4-ol ; origine : Firmenich, Switzerland. 7. [3-Oxo-2-((E)-pentyl)-cyclopentyl]-acetic acid methyl ester ; origine : Firmenich, Switzerland. 8. 3-Benzo[1,3]dioxol-5-yl-2-methyl-propionaldehydé ; origine : International Flavors and Fragrances, USA 9. 1-(2,3,8,8-Tetramethyl-1,2,3,4,5,6,7,8-octahydro-naphthalen-2-yl)-ethanone ; origine : International Flavours and Fragrances, USA. 10. 3-(4-tert-Butyl-phenyl)-2-methyl-propionaldehyde ; origine : Givaudan Switzerland. 11. 2,6-Dimethyl-hept-5-enal ; origine : Givaudan Switzerland. 12. (E)-3-Methyl-4-(2,6,6-trimethyl-cyclohex-2-enyl)-but-3-en-2-one ; origine : Firmenich, Switzerland. 13. Origine : V. Mane Fils, France. 14. 10 % dans dipropylène glycol. 15. Origine : Firmenich, Switzerland. 16. Carbonic acid (E)-hex-3-enyl ester methyl ester ; origine : International Flavors and Fragrances, USA. 17. 2,4-Dimethyl-cyclohex-3-enecarbaldehyde ; origine : International Flavors and Fragrances, USA. 18. Ethoxy-méthoxy-cyclododécane ; origine : Henkel, Allemagne. | | | |

Les essais de la composition n° 1 ont montré que les méthoxy-triméthyl-cyclododécadiènes (6a-d) sont dépourvus de la note ambrée du Boisambrène^{™} mais sont par contre plus puissants que le Boisambrène^{™} dans leurs notes boisées et ont de ce fait un impact similaire à celui du Cédramber^{™}.

Les essais de la composition n° 2 ont confirmé que les méthoxy-triméthyl-cyclododécadiènes (6a-d) sont moins ambrés et moins montants que le Boisambrène^{™}, par contre ils sont plus sombres dans les notes de fond et ils apportent des facettes de bois de cèdre, miellées et cireuses.

De plus, la perte d'intensité avec le temps semble assez linéaire dans tous les cas, sans laisser apparaître un important changement de caractéristique odorante.

Les résultats de ces évaluations montrent sans le moindre doute que le mélange de composés décrits ci-dessus présente des caractéristiques odorantes intéressantes, qui trouveront une application en particulier en parfumerie.

## Revendications

1. Composé de formule générale (I), ci-dessous : 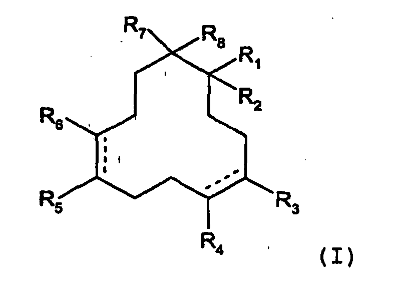 dans laquelle :
A)
a) R₄, R₅, R₇ représentent chacun un atome d'hydrogène et R₃, R₆ et R₈ représentent chacun un radical méthyle, ou
b) R₄, R₆, R₇ représentent chacun un atome d'hydrogène et R₃, R₅ et R₈ représentent chacun un radical méthyle, ou
c) R₃, R₆ et R₇ représentent chacun un atome d'hydrogène et R₄, R₅ et R₈ représentent chacun un radical méthyle,
les liaisons en pointillé sont présentes et représentent des doubles liaisons cis- ou trans-,
et R₁ représente un atome d'hydrogène et R₂ représente un groupe -OH, -OCH₃,-OC₂H₅,
les liaisons en pointillé sont absentes et R₁ représente un atome d'hydrogène et R₂ représente un groupe -OCH₃ ou -OC₂H₅,
ou
B)
R₁, R₄, R₆ représentent chacun un atome d'hydrogène et R₂, R₃ et R₅ représentent chacun un radical méthyle,
et
les liaisons en pointillé sont présentes et représentent des doubles liaisons cis- ou trans-, et R₇ représente un atome d'hydrogène et R₈ représente un groupe -OH, -OCH₃ ou - OC₂H₅,
ou
les liaisons en pointillé sont absentes et R₇ représente un atome d'hydrogène et R₈ représente un groupe -OCH₃ ou -OC₂H₅,
ledit composé étant un dérivé alcoolique ou éther.

2. Composé selon la revendication **1**, répondant à l'une des formules ci-dessous : 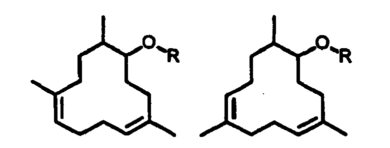 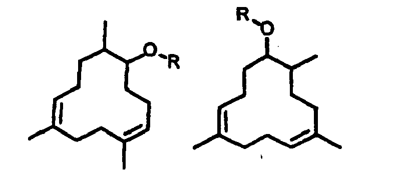 où R est un atome d'hydrogène, un groupe méthyle ou un groupe éthyle.

3. Composé selon la revendication 1, répondant à l'une des formules ci-dessous : 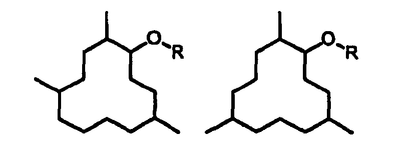 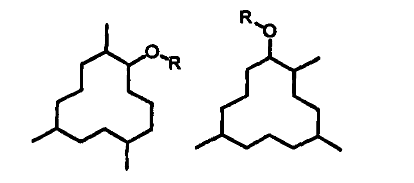 où R est un groupe méthyle ou un groupe éthyle.

4. Procédé de préparation d'un composé de formule (I) tels que défini dans les revendications 1 à 3 à partir des cétones 4a-d ci-dessous 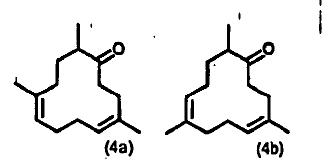 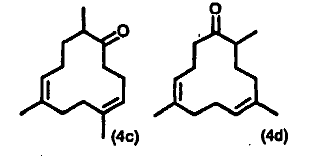 comprenant une étape de réduction des cétones en alcools, pour obtenir les alcools 5a-d suivants

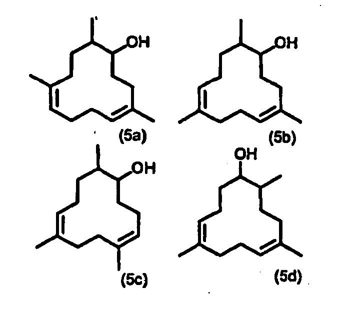

5. Procédé de préparation selon la revendication 4 d'un composé de formule (I) tels que défini dans les revendications 1 à 3, comprenant en outre une étape supplémentaire d'éthérification, pour obtenir les éthers 6a-d ou 6a'-d' suivant :

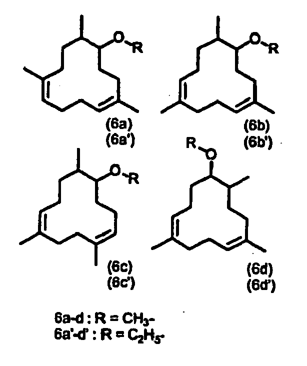

6. Procédé de préparation selon la revendication 5 d'un composé de formule (I) tels que défini dans les revendications 1 à 3, comprenant en outre une étape supplémentaire d'hydrogénation, pour obtenir les éthers saturés 7a-d ou 7a'-d' suivant :

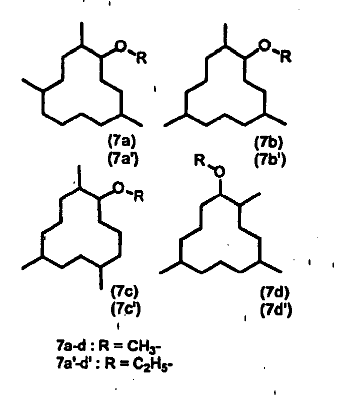

7. Procédé de préparation selon l'une quelconque des revendications **4** à **6,** dans lequel les cétones 4a-d sont obtenues par les étapes de :
- formation de dérivés chloro-nitroso à partir du 1,5,10-triméthyl-cyclododéca-1,5,9-triène (1a) et du 1,5,9-triméthyl-cyclododéca-1,5,9-triène (1b),
- transformation de ces dérivés en oximes,
- transformation réductrice des oximes en cétones en présence de Nickel de Raney, d'acétone et d'acide borique.

8. Composition **caractérisée en ce qu'**elle contient au moins un composé de formule (I), tel que défini dans l'une quelconque des revendications **1** à **3,** sous forme d'un isomère ou d'un mélange d'isomères, en particulier d'un énantiomère ou d'un mélange d'énantiomères, ou d'un mélange racémique, ou d'un diastéréoisomère ou mélange de diastéréoisomères.

9. Composition selon la revendication **8, caractérisée en ce qu'**elle contient un mélange d'au moins deux composés choisis parmi les composés 5a, 5b, 5c et/ou 5d, et préférentiellement, un mélange des quatre composés 5a, 5b 5c et 5d suivants :

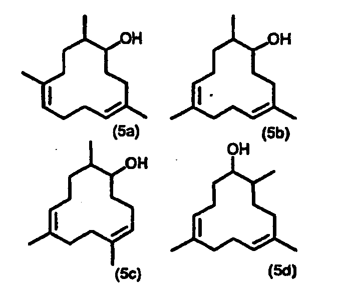

10. Composition selon la revendication **8, caractérisée en ce qu'**elle contient un mélange d'au moins deux composés choisis parmi les composés 6a, 6b, 6c et/ou 6d, et préférentiellement, un mélange des quatre composés 6a, 6b, 6c et 6d : 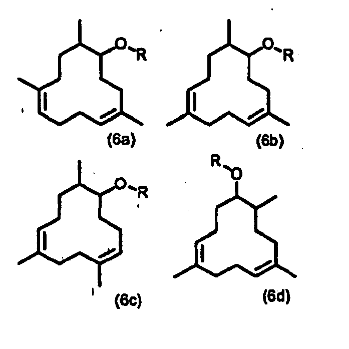 où R est un groupe méthyle.

11. Composition selon la revendication **8, caractérisée en ce qu'**elle contient un mélange d'au moins deux composés choisis parmi les composés 6a', 6b', 6c' et/ou 6d', et préférentiellement, un mélange des quatre composés 6a', 6b', 6c' et 6d' 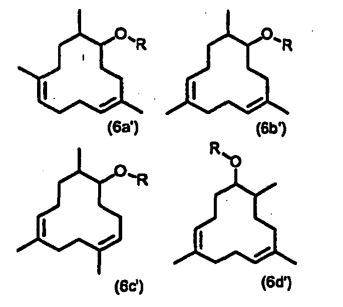 où R est un groupe éthyle.

12. Composition selon la revendication **8, caractérisée en ce qu'**elle contient un mélange d'au moins deux composés choisis parmi les composés 7a, 7b, 7c et/ou 7d, et préférentiellement, un mélange des quatre composés 7a, 7b, 7c et 7d : 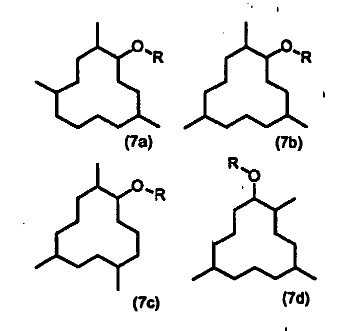 où R est un groupe méthyle.

13. Composition selon la revendication **8, caractérisée en ce qu'**elle contient un mélange d'au moins deux composés choisis parmi les composés 7a', 7b', 7c' et/ou 7d', et préférentiellement, un mélange des quatre composés 7a', 7b', 7c' et 7d', 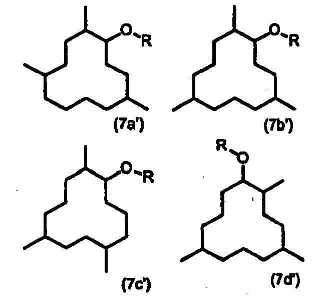 où R est un groupe éthyle.

14. Composition selon l'une quelconque des revendications **8** à **13, caractérisé en ce que** le ou lesdits composés de formule (I) sont incorporés dans ou sur un matériau support inerte ou qui peut contenir d'autres ingrédients actifs, ledit support étant notamment choisi parmi les solvants polaires, les huiles, les graisses, les solides finement divisés, les cyclodextrines, les maltodextrines, les gommes, les résines.

15. Composition de parfumerie, notamment base ou concentré parfumant, eau de Cologne, eau de toilette ou parfum, **caractérisée en ce qu'**elle comprend au moins un composé tel que défini dans l'une quelconque des revendications **1** à **3** ou une composition selon l'une quelconque des revendications **8** à **13.**

16. Composition de parfumerie selon la revendication **15, caractérisée en ce qu'**elle comprend au moins une composition telle que définie dans la revendication **10.**

17. Composition cosmétique; notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade, **caractérisée en ce qu'**elle comprend au moins un composé tel que défini dans l'une quelconque des revendications **1** à **3** ou une composition selon l'une quelconque des revendications **8** à **13.**

18. Produit d'entretien, notamment assouplissant, détergent, lessive, désodorisant d'ambiance, **caractérisé en ce qu'**il comprend au moins un composé tel que défini dans l'une quelconque des revendications **1** à **3** ou une composition selon l'une quelconque des revendications **8** à **17.**

19. Utilisation d'au moins un composé de formule (I) tel que défini dans l'une quelconque des revendications **1** à **3,** ou d'une composition selon l'une quelconque des revendications **8** à **17,** comme agent odorant ou comme agent de masquage d'odeur ou comme agent de neutralisation d'odeur.

20. Utilisation selon la revendication **19**, dans lequel le(s)dit(s) composé ou ladite composition est utilisé(e) en association avec d'autres agents odorants.

## Claims

1. A compound of general formula (I) below: 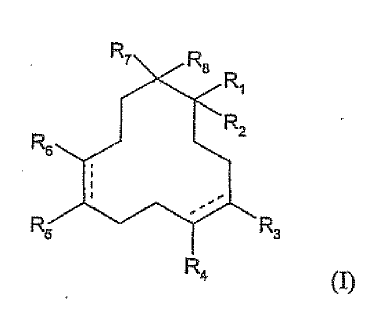 in which:
A)
a) R₄, R₅ and R₇ each represent a hydrogen atom and R₃, R₆ and R₈ each represent a methyl radical, or
b) R₄, R₆ and R₇ each represent a hydrogen atom and R₃, R₅ and R₈ each represent a methyl radical, or
c) R₃, R₆ and R₇ each represent a hydrogen atom and R₄, R₅ and R₈ each represent a methyl radical,
and
the dashed lines are present and represent cis or trans double bonds and R₁ represents a hydrogen atom and R₂ represents an -OH, -OCH₃ or -OC₂H₅ group,
or
the dashed lines are absent and R₁ represents a hydrogen atom and R₂ represents an -OCH₃ or -OC₂H₅ group,
or
B)
R₁, R₄ and R₆ each represent a hydrogen atom and R₂, R₃ and R₅ each represent a methyl radical,
and
the dashed lines are present and represent *cis* or *trans* double bonds and R₇ represents a hydrogen atom and R₈ represents an -OH, -OCH₃ or -OC₂H₅ group,
or
the dashed lines are absent and R₇ represents a hydrogen atom and R₈ represents an -OCH₃ or -OC₂H₅ group,
said compound being an alcohol or ether derivative.

2. The compound as claimed in claim 1, corresponding to one of the following formulae: 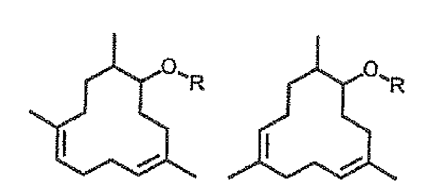 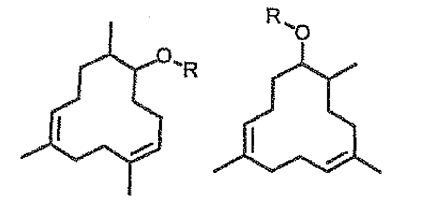 in which R is a hydrogen atom, a methyl group or an ethyl group.

3. The compound as claimed in claim 1, corresponding to one of the following formulae: 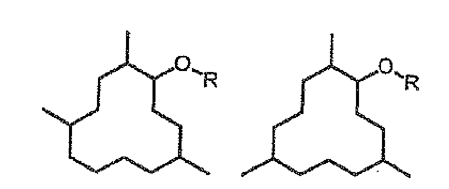 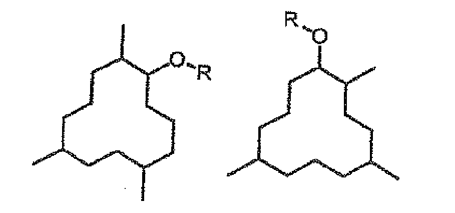 in which R is a methyl group or an ethyl group.

4. A process for preparing a compound of formula (I) as defined in claims 1 to 3, from the ketones 4a-d below 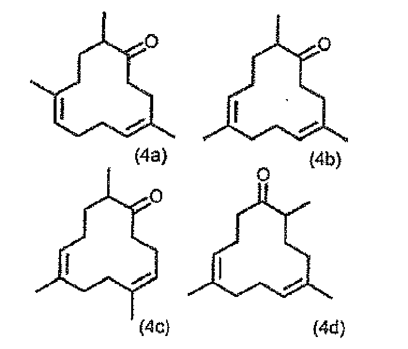 comprising a step of reducing the ketones to alcohols, to obtain the alcohols 5a-d below:

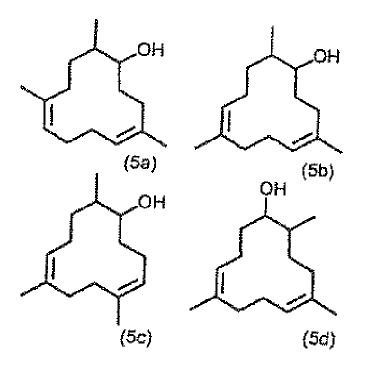

5. The preparation process as claimed in claim 4, of a compound of formula (I) as defined in claims 1 to 3, also comprising an additional step of etherification, to obtain the ethers 6a-d or 6a'-d' below:

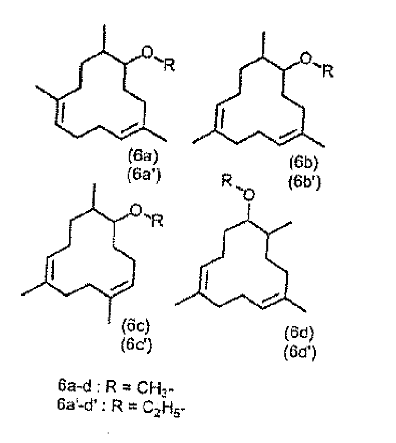

6. The preparation process as claimed in claim 5, of a compound of formula (I) as defined in claims 1 to 3, also comprising an additional step of hydrogenation, to obtain the saturated ethers 7a-d or 7a'-d' below:

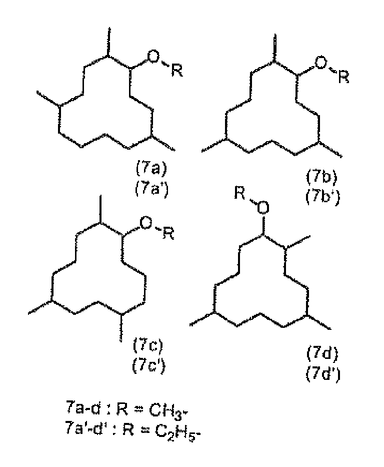

7. The preparation process as claimed in any one of claims 4 to 6, in which the ketones 4a-d are obtained via the steps of:
- formation of chloro-nitroso derivatives from 1,5,10-trimethylcyclododeca-1,5,9-triene (1a) and from 1,5,9-trimethylcyclododeca-1,5,9-triene (1b),
- conversion of these derivatives into oximes,
- reductive conversion of the oximes into ketones in the presence of Raney nickel, acetone and boric acid.

8. A composition, **characterized in that** it contains at least one compound of formula (I), as defined in any one of claims 1 to 3, in the form of an isomer or a mixture of isomers, in particular of an enantiomer or a mixture of enantiomers, or of a racemic mixture, or of a diastereoisomer or mixture of diastereoisomers.

9. The composition as claimed in claim 8, **characterized in that** it contains a mixture of at least two compounds chosen from compounds 5a, 5b, 5c and/or 5d, and preferentially a mixture of the four compounds 5a, 5b, 5c and 5d below:

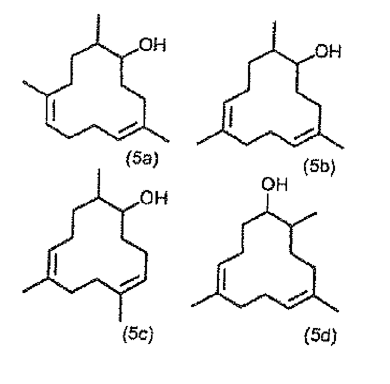

10. The composition as claimed in claim 8, **characterized in that** it contains a mixture of at least two compounds chosen from compounds 6a, 6b, 6c and/or 6d, and preferentially a mixture of the four compounds 6a, 6b, 6c and 6d: 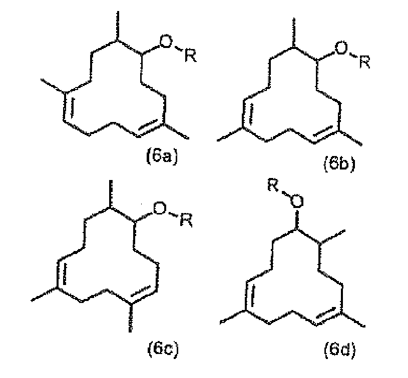 in which R is a methyl group.

11. The composition as claimed in claim 8, **characterized in that** it contains a mixture of at least two compounds chosen from compounds 6a', 6b', 6c' and/or 6d', and preferentially a mixture of the four compounds 6a', 6b', 6c' and 6d': 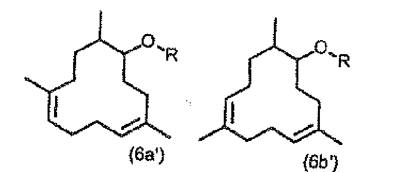 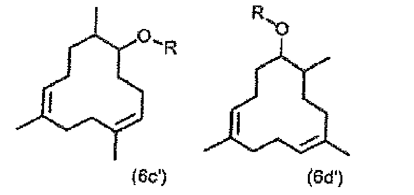 in which R is an ethyl group.

12. The composition as claimed in claim 8, **characterized in that** it contains a mixture of at least two compounds chosen from compounds 7a, 7b, 7c and/or 7d, and preferentially a mixture of the four compounds 7a, 7b, 7c and 7d: 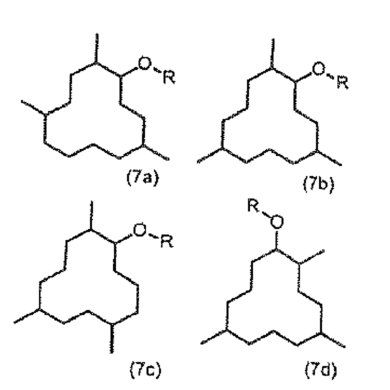 in which R is a methyl group.

13. The composition as claimed in claim 8, **characterized in that** it contains a mixture of at least two compounds chosen from compounds 7a', 7b', 7c' and/or 7d', and preferentially a mixture of the four compounds 7a', 7b', 7c' and 7d': 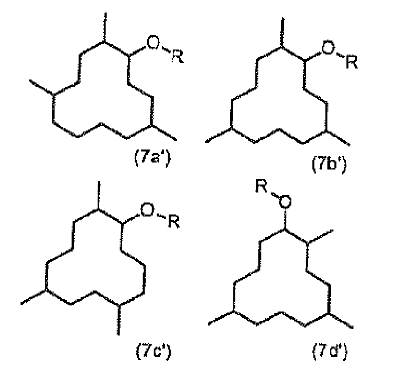 in which R is an ethyl group.

14. The composition as claimed in any one of claims 8 to 13, **characterized in that** said compound(s) of formula (I) is (are) incorporated into or onto a support material that is inert or that may contain other active ingredients, said support especially being chosen from polar solvents, oils, greases, finely divided solids, cyclodextrins, maltodextrins, gums, resins.

15. A perfumery composition, especially a fragrant base or concentrate, eau de Cologne, eau de toilette or fragrance, **characterized in that** it comprises at least one compound as defined in any one of claims 1 to 3 or a composition as claimed in any one of claims 8 to 13.

16. The perfumery composition as claimed in claim 15, **characterized in that** it comprises at least one composition as defined in claim 10.

17. A cosmetic composition, especially a face or body cream, talcum powder, hair or body oil, shampoo, hair lotion, bath salt, bath oil, shower gel, bath gel, toiletry soap, body antiperspirant, body deodorant, lotions, shaving cream, shaving soap, cream, toothpaste, mouthwash or pomade, **characterized in that** it comprises at least one compound as defined in any one of claims 1 to 3 or a composition as claimed in any one of claims 8 to 13.

18. A maintenance product, especially laundry softener, detergent, laundry washing product or ambiance deodorant, **characterized in that** it comprises at least one compound as defined in any one of claims 1 to 3 or a composition as claimed in any one of claims 8 to 17.

19. The use of at least one compound of formula (I) as defined in any one of claims 1 to 3, or of a composition as claimed in any one of claims 8 to 17, as a fragrant agent or as an odor-masking agent or as an odor-neutralizing agent.

20. The use as claimed in claim 19, in which said compound(s) or said composition is used in combination with other fragrant agents.

## Patentansprüche

1. Verbindung der nachstehenden allgemeinen Formel (I): 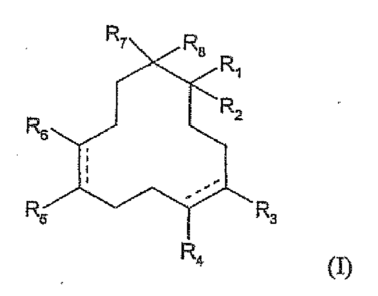 worin:
A)
a) R₄, R₅ und R₇ jeweils für ein Wasserstoffatom stehen und R₃, R₆ und R₈ jeweils für einen Methylrest stehen oder
b) R₄, R₆ und R₇ jeweils für ein Wasserstoffatom stehen und R₃, R₅ und R₈ jeweils für einen Methylrest stehen oder
c) R₃, R₆ und R₇ jeweils für ein Wasserstoffatom stehen und R₄, R₅ und R₈ jeweils für einen Methylrest stehen
und
die gestrichelten Bindungen vorhanden sind und für cis- oder trans-Doppelbindungen stehen und R₁ für ein Wasserstoffatom steht und R₂ für eine OH-, CH₃O-, C₂H₅O-Gruppe steht,
oder
die gestrichelten Bindungen fehlen und R₁ für ein Wasserstoffatom steht und R₂ für eine CH₃O- oder C₂H₅O- Gruppe steht,
oder
B)
R₁, R₄ und R₆ jeweils für ein Wasserstoffatom stehen und R₂, R₃ und R₅ jeweils für einen Methylrest stehen
und
die gestrichelten Bindungen vorhanden sind und für cis- oder trans-Doppelbindungen stehen und R₇ für ein Wasserstoffatom steht und R₈ für eine OH-, CH₃O- oder C₂H₅O-Gruppe steht
oder
die gestrichelten Bindungen fehlen und R₇ für ein Wasserstoffatom steht und R₈ für eine CH₃O- oder C₂H₅O-Gruppe steht,
wobei es sich bei der Verbindung um ein Alkohol- oder Etherderivat handelt.

2. Verbindung nach Anspruch 1 mit einer der nachstehenden Formeln: 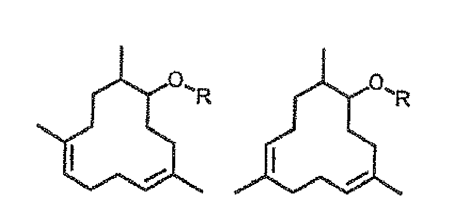 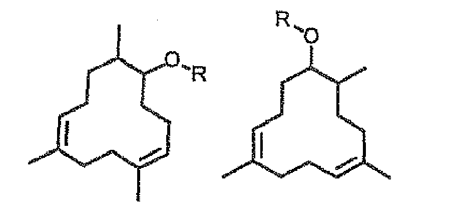 worin R für ein Wasserstoffatom, eine Methylgruppe oder eine Ethylgruppe steht.

3. Verbindung nach Anspruch 1 mit einer der nachstehenden Formeln: 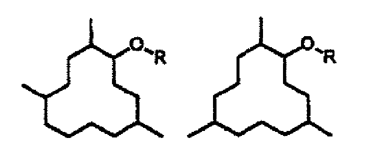 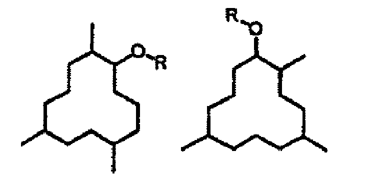 worin R für eine Methylgruppe oder eine Ethylgruppe steht.

4. Verfahren zur Herstellung einer Verbindung der Formel (I) gemäß den Ansprüchen 1 bis 3 aus den nachstehenden Ketonen 4a-d 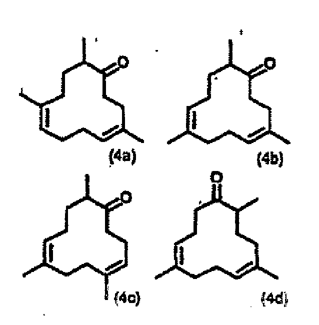 das einen Schritt der Reduktion der Ketone zu Alkoholen umfaßt, bei dem die folgenden Alkohole 5a-d erhalten werden,

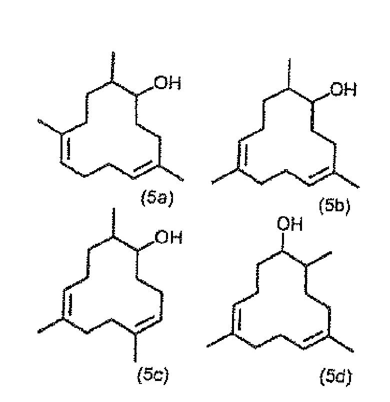

5. Verfahren nach Anspruch 4 zur Herstellung einer Verbindung der Formel (I) gemäß den Ansprüchen 1 bis 3, das außerdem einen zusätzlichen Veretherungsschritt umfaßt, bei dem die folgenden Ether 6a-d bzw. 6a'-d' erhalten werden,

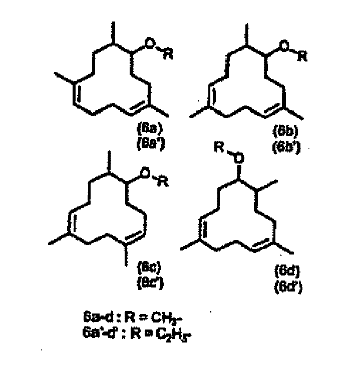

6. Verfahren nach Anspruch 5 zur Herstellung einer Verbindung der Formel (I) gemäß den Ansprüchen 1 bis 3, das außerdem einen zusätzlichen Hydrierungsschritt umfaßt, bei dem die folgenden gesättigten Ether 7a-d bzw. 7a'-d' erhalten werden,

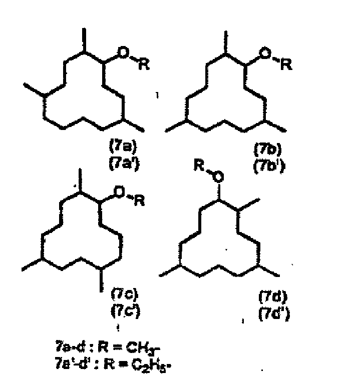

7. Herstellungsverfahren nach einem der Ansprüche 4 bis 6, bei dem man die Ketone 4a-d durch die folgenden Schritte erhält:
- Bildung von Chlornitrosoderivaten aus 1,5,10-Trimethylcyclododeca-1,5,9-trien (1 a) und 1,5,9-Trimethylcyclododeca-1,5,9-trien (1b),
- Umwandlung dieser Derivate in Oxime,
- reduzierende Umwandlung der Oxime in Ketone in Gegenwart von Raney-Nickel, Aceton und Borsäure.

8. Zusammensetzung, **dadurch gekennzeichnet, daß** sie mindestens eine Verbindung der Formel (I) gemäß einem der Ansprüche 1 bis 3 in Form eines Isomers oder Isomerengemischs, insbesondere eines Enantiomers oder eines Enantiomerengemischs, oder eines Racemats oder eines Diastereoisomers oder eines Diastereoisomerengemischs enthält.

9. Zusammensetzung nach Anspruch 8, **dadurch gekennzeichnet, daß** sie ein Gemisch von mindestens zwei Verbindungen, die unter den Verbindungen 5a, 5b, 5c und/oder 5d ausgewählt sind, und vorzugsweise ein Gemisch der vier folgenden Verbindungen 5a, 5b, 5c und 5d enthält:

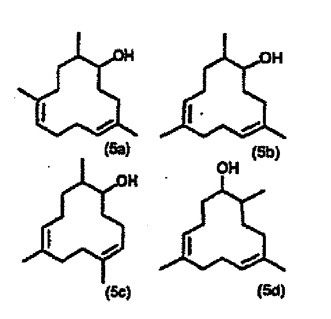

10. Zusammensetzung nach Anspruch 8, **dadurch gekennzeichnet, daß** sie ein Gemisch von mindestens zwei Verbindungen, die unter den Verbindungen 6a, 6b, 6c und/oder 6d ausgewählt sind, und vorzugsweise ein Gemisch der vier folgenden Verbindungen 6a, 6b, 6c und 6d 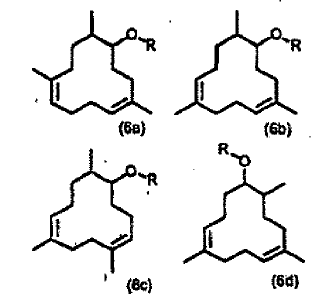 worin R für eine Methylgruppe steht, enthält.

11. Zusammensetzung nach Anspruch 8, **dadurch gekennzeichnet, daß** sie ein Gemisch von mindestens zwei Verbindungen, die unter den Verbindungen 6a', 6b', 6c' und/oder 6d' ausgewählt sind, und vorzugsweise ein Gemisch der vier folgenden Verbindungen 6a', 6b', 6c' und 6d' 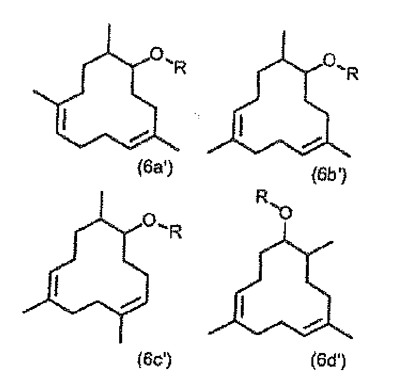 worin R für eine Ethylgruppe steht, enthält.

12. Zusammensetzung nach Anspruch 8, **dadurch gekennzeichnet, daß** sie ein Gemisch von mindestens zwei Verbindungen, die unter den Verbindungen 7a, 7b, 7c und/oder 7d ausgewählt sind, und vorzugsweise ein Gemisch der vier folgenden Verbindungen 7a, 7b, 7c und 7d 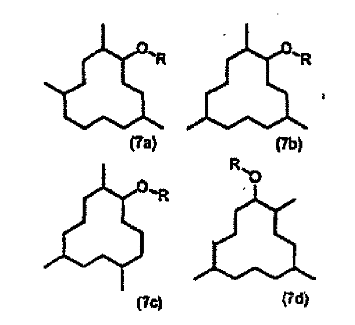 worin R für eine Methylgruppe steht, enthält.

13. Zusammensetzung nach Anspruch 8, **dadurch gekennzeichnet, daß** sie ein Gemisch von mindestens zwei Verbindungen, die unter den Verbindungen 7a', 7b', 7c' und/oder 7d' ausgewählt sind, und vorzugsweise ein Gemisch der vier folgenden Verbindungen 7a', 7b', 7c' und 7d' 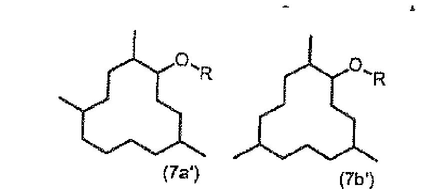 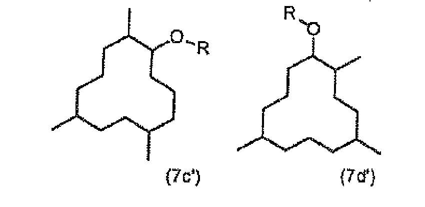 worin R für eine Ethylgruppe steht, enthält.

14. Zusammensetzung nach einem der Ansprüche 8 bis 13, **dadurch gekennzeichnet, daß** die Verbindung bzw. die Verbindungen der Formel (I) in oder auf einem inerten oder andere Wirkstoffe enthaltenden Trägermaterial inkorporiert ist bzw. sind, wobei der Träger insbesondere unter polaren Lösungsmitteln, Ölen, Fetten, feinteiligen Feststoffen, Cyclodextrinen, Maltodextrinen, Gummen und Harzen ausgewählt ist.

15. Parfümeriezusammensetzung, insbesondere Parfümbasis oder - konzentrat, Eau de Cologne, Eau de Toilette oder Parfüm, **dadurch gekennzeichnet, daß** sie mindestens eine Verbindung gemäß einem der Ansprüche 1 bis 3 oder eine Zusammensetzung nach einem der Ansprüche 8 bis 13 umfaßt.

16. Parfümeriezusammensetzung nach Anspruch 15, **dadurch gekennzeichnet, daß** sie mindestens eine Zusammensetzung gemäß Anspruch 10 umfaßt.

17. Kosmetische Zusammensetzung, insbesondere Gesichts- oder Körpercreme, Talkumpulver, Haar- oder Körperöl, Shampoo, Haarlotion, Badesalz, Badeöl, Duschgel, Badegel, Toilettenseife, Körperantitranspirant, Körperdeodorant, Lotionen, Rasiercreme, Rasierseife, Creme, Zahnpasta, Mundwasser oder Pomade, **dadurch gekennzeichnet, daß** sie mindestens eine Verbindung gemäß einem der Ansprüche 1 bis 3 oder eine Zusammensetzung nach einem der Ansprüche 8 bis 13 umfaßt.

18. Pflegeprodukt, insbesondere geschmeidigmachendes Produkt, Reinigungsmittel, Waschmittel oder Umweltdeodorant, **dadurch gekennzeichnet, daß** es mindestens eine Verbindung gemäß einem der Ansprüche 1 bis 3 oder eine Zusammensetzung nach einem der Ansprüche 8 bis 17 umfaßt.

19. Verwendung mindestens einer Verbindung der Formel (I) gemäß einem der Ansprüche 1 bis 3 oder einer Zusammensetzung nach einem der Ansprüche 8 bis 17 als Riechstoff oder als Geruchsmaskierungsmittel oder als Geruchsneutralisierungsmittel.

20. Verwendung nach Anspruch 19, wobei die Verbindung bzw. die Verbindungen oder die Zusammensetzung in Kombination mit anderen Riechstoffen verwendet wird bzw. werden.
